# EUROPEAN PATENT APPLICATION

(11) **EP 1 186 592 A1**
(43) Date of publication of application: **13.03.2002**
(21) Application number: 01121523.3
(22) Date of filing: 08.09.2001
(51) Int. Cl.: C07C 67/327, C07C 67/03, C07C 69/54

(54) **Production of methacrylates**

(30) Priority: 11.09.2000 JP 2000275018
(71) Applicant: MITSUBISHI GAS CHEMICAL COMPANY, LTD., Tokyo, 100 (JP)
(72) Inventor: Hidaka, Toshio, c/oMitsubishi Gas Chem. Comp. Inc., Tsukuba-shi, Ibaraki (JP); Sato, Yoshifumi, c/oMitsubishi Gas Chem. Comp. Inc, Tsukuba-shi, Ibaraki (JP); Taniguchi, Mitsugu, c/oMitsubishi Gas Chem Co. Inc, Tsukuba-shi, Ibaraki (JP)
(74) Representative: Gille Hrabal Struck Neidlein Prop Roos

(57) **Abstract**

Methacrylates, particularly, esters of methacrylic acid with an alcohol having two or more carbon atoms or a polyhydric alcohol are produced by a one-stage reaction of α-hydroxyisobutyric acid and/or its ester with an alcoholic compound in the presence of a solid catalyst.

## Description

### BACKGROUND OF THE INVENTION

### 1. Field of the Invention

The present invention relates to a method for producing methacrylates (methacrylic esters). The methacrylates are industrially very important compounds as the starting material for producing synthetic resins and synthetic fibers.

### 2. Description of the Prior Art

The methacrylates have been produced mainly by the esterification reaction of methacrylic acid by an alcohol or the ester exchange reaction of a methacrylate by an alcohol. Esters of α,β-unsaturated carboxylic acid such as acrylic acid and methacrylic acid have been generally produced by liquid-phase or vapor-phase esterification or ester exchange reaction in the presence of a catalyst selected from sulfuric acid, phosphoric acid, p-toluenesulfonic acid and a solid acid such as a strongly acidic ion-exchange resin, a solid phosphoric acid and silica-titania.

As the liquid phase method, proposed are, for example, the production of hydroxyalkyl acrylates in the presence of a mineral acid such as sulfuric acid and hydrochloric acid as the esterification catalyst (DE 1518572), and a method of using di-n-butyl tin oxide, stannoxane or distannoxane as the ester exchange catalyst (Japanese Patent Application Laid-Open Nos. 46-39848, 52-153913, and 7-97387). As the vapor phase method, proposed are methods using a solid phosphoric acid catalyst (Japanese Patent Publication No. 42-5222), a molybdenum sulfide catalyst (Japanese Patent Publication No. 44-9885), and a silica-titania catalyst (Japanese Patent Publication No. 55-33702). However, the above liquid and vapor phase methods involve problems of the reduction in the selectivity and productivity due to side reactions such as addition reaction to double bond, dehydrating condensation of alcohols and formation of dimer ester by intermolecular dehydration. Since mainly carried out in batch wise manner, the liquid phase method is not suited for mass production.

Methacrylic acid and methacrylic esters can be also produced from hydroxyisobutyric acid and hydroxyisobutyric esters. For example, Japanese Patent Application Laid-Open No. 8-188555 proposes a method of producing methyl methacrylate from methyl α-hydroxyisobutyrate using a faujasite zeolite as a vapor-phase dehydration catalyst. This method is an ammonium sulfate-free, excellent method in which acetone cyanohydrin is converted to methyl α-hydroxyisobutyrate which is then dehydrated into methyl methacrylate. However, known industrially applicable methods of producing the methacrylates from hydroxyisobutyric acid or its esters are, except for the production of methyl methacrylate, still insufficient in the selectivity and yield. Therefore, the known methods are unsuitable for the industrial production of a variety of methacrylates from hydroxyisobutyric acid and its esters.

### SUMMARY OF THE INVENTION

An object of the present invention is to provide an easy and simple method for producing industrially important methacrylates from α-hydroxyisobutyric acid or a hydroxyisobutyrate such as methyl α-hydroxyisobutyrate, particularly, to provide a single-stage method for producing methacrylates which have been difficult to produce by known methods, i.e., esters of methacrylic acid with an alcohol having two or more carbon atoms or an polyhydric alcohol.

As a result of extensive study in view of solving the problems in the prior art, the inventors have found that the methacrylates are easily produced by the reaction of hydroxyisobutyric acid and/or a hydroxyisobutyric ester with an alcoholic compound in the presence of a solid catalyst. The invention has been accomplished on the basis of this finding.

Thus, the present invention provides a method for producing methacrylates, which comprises a step of reacting hydroxyisobutyric acid and/or a hydroxyisobutyric ester with an alcoholic compound in the presence of a solid catalyst.

### DETAILED DESCRIPTION OF THE INVENTION

First starting material used in the present invention is selected from α-hydroxyisobutyric acid, β-hydroxyisobutyric acid and α- or β-hydroxyisobutyric esters. The α- or β-hydroxyisobutyric ester may be methyl ester, ethyl ester, n-propyl ester, isopropyl ester, allyl ester, glycidyl ester, n-butyl ester, isobutyl ester, sec-butyl ester, tert-butyl ester, hexyl ester, octyl ester, 2-ethylhexyl ester, lauryl ester, stearyl ester, cyclohexyl ester, benzyl ester, 2-hydroxyethyl ester, 2-hydroxypropyl ester, 3-hydroxypropyl ester, and 4-hydroxybutyl ester. In view of industrially easy availability, particularly preferred are α-hydroxyisobutyric acid, β-hydroxyisobutyric acid, methyl α-hydroxyisobutyrate, methyl β-hydroxyisobutyrate, ethyl α-hydroxyisobutyrate, ethyl β-hydroxyisobutyrate, 2-ethylhexyl α-hydroxyisobutyrate, and 2-ethylhexyl β-hydroxyisobutyrate. These compounds may be used singly or in combination of two or more. The first starting material may contain a small amount of methacrylic acid or a methacrylic ester because these compounds adversely affect the reaction little.

Second starting material is an alcoholic compound. When the hydroxyisobutyric ester is used as the first starting material, the alcoholic compound for the second starting material should be different from the alcohol constituting the ester moiety of the hydroxyisobutyric ester. The alcoholic compound is alcohol, glycol or polyol each having 1 to 24, preferably 2 to 9 carbon atoms. Example thereof include methanol, ethanol, n-propyl alcohol, isopropyl alcohol, allyl alcohol, n-butyl alcohol, isobutyl alcohol, tert-butyl alcohol, hexanol, octanol, 2-ethylhexanol, lauryl alcohol, stearyl alcohol, cyclohexanol, benzyl alcohol, ethylene glycol, 1,3-propanediol, and 1,4-butanediol, with methanol, ethanol, propanols, butanols, ally alcohol, ethylene glycol and cyclohexanol being preferred, and butanols, ally alcohol, ethylene glycol and cyclohexanol being more preferred. The above alcoholic compounds may be used singly or in combination of two or more.

Example of the solid catalyst used in the present invention includes a mesoporous compound such as silica, alumina, titania, zirconia, silicalite, heteropolyacid and mosoporous silica such as MCM-41; a crystalline aluminosilicate, a crystalline silicoaluminophosphate; and naturally occurring clay mineral such as kaolinite, montmorillonite, sepiolite and mica with silica, silicalite, crystalline aluminosilicate, and crystalline silicoaluminophosphate being preferred.

The solid catalyst preferably supports cesium as an effective ingredient in the form of carbonate, hydroxide or nitrate such as cesium carbonate, cesium hydroxide and cesium nitrate. Since cesium is difficult to exist in the form of oxide even after calcinations, it is preferably supported on the solid catalyst in the form of carbonate, hydroxide or nitrate as mentioned above.

Cesium is supported on the solid catalyst by any method selected from an impregnation method, a coprecipitation method, a sol-gel method, an ion-exchange method, a physical blend method, a thermal decomposition method, a chemical deposition method, a mechanochemical method, and a synthetic method in which, for example, a cesium compound is added in the production of molecular sieve. The supporting amount of cesium is not specifically limited, and preferably 0.1 to 20% by weight, in terms of metallic cesium, based on the solid catalyst.

The catalytic activity and the selectivity can be further improved if another metal element is combinedly used with cesium. As the metal element for such purposes, preferred is at least one metal selected from the group consisting of Na, K, Rb, Mg Ca, La, Ce, Sr, Ba, Y, Ti, Zr, V, Nb, Ta, Cr, Fe, Ru, Co, Ir, Ni, Pd, Pt, Cu, Ge, In, Sn and Sb. The cesium compound and a compound of the metal selected from the above group are supported on the solid catalyst, for example, by an impregnation method, a coprecipitation method, an ion-exchange method, a physical blend method, a chemical deposition method, a thermal decomposition method, or a method where a precursor compound is added and then calcined. The supporting amount of the additional metal is preferably 0.01 to 50% by weight, in terms of metal basis, based on the solid catalyst.

The solid catalyst, which may optionally support cesium alone or in combination with the additional metal, may be used in the reaction directly or after calcined generally at 300 to 700 °C for 1 to 24 hours using a binder such as silica, alumina, titania and various clay minerals and then formed into a shaped article by a compression molding or a punching tablet molding.

The production method of the present invention may be carried out in either vapor phase or liquid phase, and in any manner of batch wise manner, semi-batch wise manner and continuous manner, each at ordinary pressure or increased pressure. Therefore, the reaction method is selected by considering the types and properties of the starting materials, the types of reaction apparatus, etc. Generally, a vapor-phase fixed bed flow method is preferably employed because of its easy operation.

In a liquid-phase batch wise reaction, for example, the hydroxyisobutyric acid or its ester and the alcoholic compound are reacted in a solvent or without using the solvent in the presence of the solid catalyst in a molar ratio of 1:0.1 to 1:10 at room temperature to 200 °C for 1 to 24 hours. The use of solvent is not critical, but if desired, usable are a hydrocarbon such as hexane and heptane and an aromatic hydrocarbon such as benzene, toluene and xylene. The solid catalyst is used preferably in an amount of 0.1 to 50% by weight based on the mixture of the starting materials.

In a vapor-phase flow method, a 1:0.1 to 1:10 (by mol) mixed gas of the hydroxyisobutyric acid or its ester and the alcoholic compound is fed into a reactor having the solid catalyst disposed in a gas hourly space velocity of 10 to 10,000 h⁻¹ at 200 to 400 °C under 0.01 to 1 MPa.

In any of the above methods, the reaction of hydroxyisobutyric acid and/or its ester with the alcoholic compound proceeds in the presence of the solid catalyst, thereby producing methacrylates, particularly, methacrylates having a C2-C12 ester moiety in a single stage.

The present invention will be explained in more detail by reference to the following example which should not be construed to limit the scope of the present invention.

In the following examples and comparative examples, the reaction was carried out in a flow reaction apparatus equipped with tanks for storing the materials, pumps for feeding the materials, a mass flow controller for feeding inert gas, a quartz reaction tube of 13 mm of inner diameter and 250 mm of length, and a sampling tank. After 2 to 4 h when the reaction reached a stationary state, the reaction liquid was sampled over about one hour. The sampled liquid was analyzed by an FID gas chromatograph to determined the distribution of the products.

### Preparation of Solid Catalyst

### Catalyst 1

After stirring 64.5 g of silica sol and 5.76 g of a 10 wt % aqueous cesium nitrate, the resultant mixture was adjusted to pH 6.7-7.3 by concentrated nitric acid. The mixture was made into gel form by adding 0.56 g of a 10 wt % aqueous ammonium nitrate. The gel was ground and formed into granulates, which were then calcined at 500 °C for 4 h, thereby preparing Catalyst 1. The result of thermal analysis showed the occurrence of no oxide of cesium and the existence of nitrate ion.

### Catalyst 2

After subjecting 20 g of a commercially available silicalite (Si/Al molar ratio = 300, MFI 300 available from UOP Co., Ltd.) to ion-exchange treatment with three portions of 500 mL of a 10 wt % aqueous cesium nitrate, the resultant product was dried at 120 °C for 2 h and calcined at 500 °C for 4 h, thereby preparing Catalyst 2.

### Catalyst 3

Under stirring, 20 g of a commercially available silicalite (Si/Al molar ratio = 300, MFI 300 available from UOP Co., Ltd.) was mixed with 2 g of cesium carbonate, 0.5 g of titanium oxide and 40 g of water. The resultant mixture was dried at 120 °C for 2 h and calcined at 500 °C for 4 h, thereby preparing Catalyst 3.

### Catalyst 4

A mixture of 40 g of a 10 wt % aqueous cesium carbonate and 25 g of silica gel was allowed to stand for 2 h. After removing the water by evaporation under reduced pressure, the mixture was dried at 120 °C for 18 h, thereby preparing Catalyst 4.

### EXAMPLE 1

Catalyst 1 (3 mL) was packed into a reaction tube, to which was fed a 1:6 (by mol) mixed gas of α-hydroxyisobutyric acid (HBA) and ethylene glycol (HG) at a gas hourly space velocity (GHSV) of 500 h⁻¹ to synthesize 2-hydroxyethyl methacrylate (2-HEMA) at 320 °C under 0.1 MPa. The results of the reaction are shown below.
Conversion of HBA: 98.9 mol%
Yield of 2-HEMA: 85.9 mol%

### EXAMPLE 2

The procedure of Example 1 was repeated except for using Catalyst 2. The results of the reaction are shown below.
Conversion of HBA: 98.6 mol%
Yield of 2-HEMA: 78.9 mol%

### EXAMPLE 3

The procedure of Example 1 was repeated except for using methyl α-hydroxyisobutyrate (HBM) in place of α-hydroxyisobutyric acid and changing the reaction temperature to 330 °C. The results of the reaction are shown below.
Conversion of HBM:99.6 mol%
Yield of 2-HEMA: 62.9 mol%

### EXAMPLE 4

The procedure of Example 1 was repeated except for using cyclohexanol (CH) in place of ethylene glycol and changing the reaction temperature to 350 °C, thereby producing cyclohexyl methacrylate (CHM). The results of the reaction are shown below.
Conversion of HBA: 100 mol%
Yield of CHM: 58.4 mol%

### EXAMPLE 5

The procedure of Example 1 was repeated except for using 2-ethylhexyl α-hydroxyisobutyrate (HBO) in place of α-hydroxyisobutyric acid and changing the reaction temperature to 340 °C. The results of the reaction are shown below.
Conversion of HBO: 98.1 mol%
Yield of 2-HEMA: 58.7 mol%

### EXAMPLE 6

The procedure of Example 1 was repeated except for using Catalyst 2 in place of Catalyst 1 and using ethyl α-hydroxyisobutyrate (HBE) in place of α-hydroxyisobutyric acid. The results of the reaction are shown below. Conversion of HBE: 94.0 mol%
Yield of 2-HEMA: 54.9 mol%

### EXAMPLE 7

The procedure of Example 1 was repeated except for using n-butyl alcohol (NB) in place of ethylene glycol to produce n-butyl methacrylate (NBA). The results of the reaction are shown below.
Conversion of HBA: 96.8 mol%
Yield of NBA: 42.9 mol%

### EXAMPLE 8

The procedure of Example 1 was repeated except for using Catalyst 3 in place of Catalyst 1. The results of the reaction are shown below.
Conversion of HBA: 99.6 mol%
Yield of 2-HEMA: 90.4 mol%

### EXAMPLE 9

The procedure of Example 1 was repeated except for using a 90 wt % aqueous α-hydroxyisobutyric acid in place of α-hydroxyisobutyric acid. The results of the reaction are shown below.
Conversion of HBA: 99.5 mol%
Yield of 2-HEMA: 89.2 mol%

### EXAMPLE 10

The procedure of Example 1 was repeated except for using Catalyst 4 in place of Catalyst 1. The results of the reaction are shown below.
Conversion of HBA: 99.6 mol%
Yield of 2-HEMA: 88.0 mol%

### EXAMPLE 11

The procedure for preparing Catalyst 1 was repeated except for using sodium carbonate in place of cesium nitrate, thereby preparing Catalyst 5. The procedure of Example 1 was repeated except for using Catalyst 5 in place of Catalyst 1. The results of the reaction are shown below.
Conversion of HBA: 97.4 mol%
Yield of 2-HEMA: 23.4 mol%

### EXAMPLE 12

The procedure of Example 1 was repeated except for using Na-substituted zeolite X (MS13X available from Aldrich Chemical Co., Inc.) as Catalyst 6 in place of Catalyst 1 and changing the reaction temperature to 300 °C. The results of the reaction are shown below.
Conversion of HBA: 98.3 mol%
Yield of 2-HEMA: 38.0 mol%

### EXAMPLE 13

The procedure of Example 1 was repeated except for using Na-substituted zeolite Y (HSZ-320NAA available from Tosho Corporation) as Catalyst 7 in place of Catalyst 1, and changing the reaction temperature to 300 °C and the molar ratio of the starting mixture to 1:4. The results of the reaction are shown below.
Conversion of HBA: 98.4 mol%
Yield of 2-HEMA: 9.4 mol%

The results of Examples 1 to 13 are collectively shown in Table 1.

**Table 1**

| | Catalyst | Starting Materials | Reaction Temperature (°C) | Conversion (mol%) | Yield (mol%) | Products |
|---|---|---|---|---|---|---|
| Examples | | | | | | |
| 1 | Catalyst 1 | HBA/EG | 320 | 98.9 | 85.9 | 2-HEMA |
| 2 | Catalyst 2 | HBA/EG | 320 | 98.6 | 78.9 | 2-HEMA |
| 3 | Catalyst 1 | HBM/EG | 330 | 99.6 | 62.9 | 2-HEMA |
| 4 | Catalyst 1 | HBA/CH | 350 | 100 | 58.4 | CHM |
| 5 | Catalyst 2 | HBO/EG | 340 | 98.1 | 58.7 | 2-HEMA |
| 6 | Catalyst 2 | HBE/EG | 320 | 94.0 | 54.9 | 2-HEMA |
| 7 | Catalyst 1 | HBA/NB | 320 | 96.8 | 42.9 | NBA |
| 8 | Catalyst 3 | HBA/EG | 320 | 99.6 | 90.4 | 2-HEMA |
| 9 | Catalyst 1 | HBA/EG | 320 | 99.5 | 89.2 | 2-HEMA |
| 10 | Catalyst 4 | HBA/EG | 320 | 99.6 | 88.0 | 2-HEMA |
| 11 | Catalyst 5 | HBA/EG | 320 | 97.4 | 23.4 | 2-HEMA |
| 12 | Catalyst 6 | HBA/EG | 300 | 98.3 | 38.0 | 2-HEMA |
| 13 | Catalyst 7 | HBA/EG | 300 | 98.4 | 9.4 | 2-HEMA |

As described above, the production method of the present invention produces industrially important methacrylates, particularly, esters of methacrylic acid with an alcohol having two or more carbon atoms or a polyhydric alcohol in easy and simple manner by the reaction of hydroxyisobutyric acid and/or its ester with the alcoholic compound. Therefore, the present invention is industrially of great significance.

## Claims

1. A method for producing methacrylates, which comprises a step of reacting hydroxyisobutyric acid and/or a hydroxyisobutyric ester with an alcoholic compound in the presence of a solid catalyst.

2. The method according to claim 1, wherein the solid catalyst is at least one substance selected from the group consisting of silica, alumina, titania, zirconia silicalite, heteropolyacid, mesoporous silica, a crystalline aluminosilicate, a crystalline silicoaluminophosphate, kaolinite, montmorillonite, sepiolite, and mica.

3. The method according to claim 1 or 2, wherein the solid catalyst is silica, silicalite, the crystalline aluminosilicate, or the crystalline silicoaluminophosphate.

4. The method according to according to any one of claims 1 to 3, wherein the hydroxyisobutyric acid is α-hydroxyisobutyric acid or β-hydroxyisobutyric acid.

5. The method according to any one of claims 1 to 3, wherein the hydroxyisobutyric ester is at least one ester selected from the group consisting of methyl ester, ethyl ester, n-propyl ester, isopropyl ester, allyl ester, glycidyl ester, n-butyl ester, isobutyl ester, sec-butyl ester, tert-butyl ester, hexyl ester, octyl ester, 2-ethylhexyl ester, lauryl ester, stearyl ester, cyclohexyl ester, benzyl ester, 2-hydroxyethyl ester, 2-hydroxypropyl ester, 3-hydroxypropyl ester, and 4-hydroxybutyl ester of α-hydroxyisobutyric acid or β-hydroxyisobutyric acid.

6. The method according to any one of claims 1 to 3, wherein the hydroxyisobutyric acid and/or a hydroxyisobutyric ester is at least one compound selected from the group consisting of α-hydroxyisobutyric acid, β-hydroxyisobutyric acid, methyl α-hydroxyisobutyrate, methyl β-hydroxyisobutyrate, ethyl α-hydroxyisobutyrate, ethyl β-hydroxyisobutyrate, 2-ethylhexyl α-hydroxyisobutyrate, and 2-ethylhexyl β-hydroxyisobutyrate.

7. The method according to any one of claims 1 to 6, wherein the alcoholic compound is alcohol, glycol or polyol each having 1 to 24 carbon atoms.

8. The method according to any one of claims 1 to 7, wherein the alcoholic compound is at least one compound selected from the group consisting of methanol, ethanol, n-propyl alcohol, isopropyl alcohol, allyl alcohol, n-butyl alcohol, isobutyl alcohol, tert-butyl alcohol, hexanol, octanol, 2-ethylhexanol, lauryl alcohol, stearyl alcohol, cyclohexanol, benzyl alcohol, ethylene glycol, 1,3-propanediol, and 1,4-butanediol.

9. The method according to any one of claims 1 to 8, wherein the alcoholic compound is at least one compound selected from the group consisting of methanol, ethanol, propanols, butanols, allyl alcohol, ethylene glycol, and cyclohexanol.

10. The method according to any one of claims 1 to 9, wherein the alcoholic compound is butanols, ally alcohol, ethylene glycol, or cyclohexanol.

11. The method according to any one of claims 1 to 10, wherein the alcoholic compound is ethylene glycol.

12. The method according to any one of claims 1 to 11, wherein the solid catalyst supports cesium.

13. The method according to claim 12, wherein cesium is supported in the form of carbonate, hydroxide or nitrate.

14. The method according to claim 12 or 13, wherein cesium is supported in an amount of 0.1 to 20% by weight based on the solid catalyst.

15. The method according to any one of claims 1 to 14, wherein the reaction between hydroxyisobutyric acid and/or a hydroxyisobutyric ester with the alcoholic compound is carried out in vapor phase at 200 to 400 °C.

16. The method according to claim 15, wherein a mixture of hydroxyisobutyric acid and/or a hydroxyisobutyric ester and the alcoholic compound is brought into contact with the solid catalyst at a gas hourly space velocity of 10 to 10,000 h⁻¹.

17. The method according to any one of claims 1 to 16, wherein the methacrylate is 2-hydroxyethyl methacrylate, cyclohexyl methacrylate or n-butyl methacrylate.

18. The method according to any one of claims 1 to 17, wherein the methacrylate is produced in one-stage reaction between hydroxyisobutyric acid and/or a hydroxyisobutyric ester with the alcoholic compound.
